# EUROPEAN PATENT APPLICATION

(11) **EP 4 467 559 A1**
(43) Date of publication of application: **27.11.2024**
(21) Application number: 23742808.1
(22) Date of filing: 13.01.2023
(51) Int. Cl.: C07J 9/00, C07D 455/03, A61K 31/575, A61K 31/4375, A61P 1/16

(54) **CRYSTAL FORM OF HIGH-STABILITY NOR-URSODEOXYCHOLIC BERBERINE SALT, AND PREPARATION METHOD THEREFOR**

(30) Priority: 20.01.2022 CN 202210067237; 28.03.2022 CN 202210313183
(71) Applicant: Chengdu Biobel Biotechnology Co., Ltd., Chengdu, Sichuan 610041 (CN)
(72) Inventor: GUANG, Bing, Chengdu, Sichuan 610041 (CN); DONG, Renhan, Chengdu, Sichuan 610041 (CN); ZHAN, Wei, Chengdu, Sichuan 610041 (CN); QIN, Chuanjun, Chengdu, Sichuan 610041 (CN); GUANG, Fengming, Chengdu, Sichuan 610041 (CN)
(74) Representative: Altmann Stößel Dick Patentanwälte PartG mbB
(86) International application number: PCT/CN2023/072039
(87) International publication number: WO 2023/138493

(57) **Abstract**

Disclosed are a crystalline form of a high-stability nor-ursodeoxycholic berberine salt and a preparation method therefor, while relate to the field of pharmaceutical chemistry. The crystalline form has the following structural formula. In X-ray powder diffraction of the crystalline form, a 2θ diffraction angle has characteristic peaks at 3.54+/-0.2 degrees, 7.13+/-0.2 degrees, 13.14+/-0.2 degrees, 15.95+/-0.2 degrees, 16.40+/-0.2 degrees, 18.64+/-0.2 degrees, 24.90+/-0.2 degrees, 25.76+/-0.2 degrees and 26.30+/-0.2 degrees. Compared with other crystalline forms of the nor-ursodeoxycholic berberine salt, the crystalline form has more excellent stability, and has a good application prospect in the preparation of high-quality solid chemical drugs.

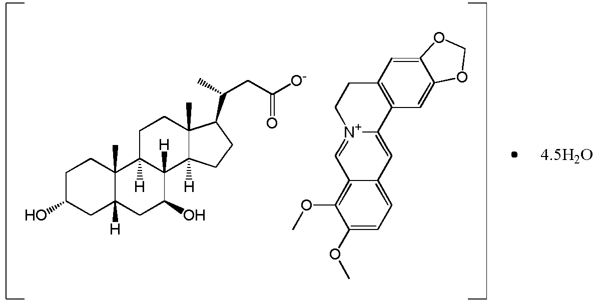

## Description

This application claims the benefit of priority to Application No. CN202210313183.7, filed on March 28, 2022 in China National Intellectual Property Administration (CNIPA) and entitled "Crystal form of high-stability nor-ursodeoxycholic berberine salt, and preparation method therefor", as well as Application No. CN202210067237.6 filed on January 20, 2022 in CNIPA, the entire contents of which are incorporated by reference herein.

### Technology of the invention

The present invention belongs to the field of pharmaceutical chemistry, and specifically relates to a crystalline form of high-stability nor-ursodeoxycholic berberine salt, and a preparation method therefor.

### Background of the invention

Fatty liver, as a common liver disease, refers to the fat accumulation in hepatocytes caused by various reasons. When the fat content exceeds 5% of the liver weight (wet weight) or histologically exceeds 30% of the liver parenchyma, the liver is called fatty liver. Fatty liver can be further divided into alcoholic fatty liver disease (AFLA) or non-alcoholic fatty liver disease (NAFLA). The latter also includes obese fatty liver, malnutrition-associated fatty liver, drug-induced fatty liver, acute fatty liver of pregnancy (AFLP), diabetes fatty liver and so on. Fatty liver can be divided into three stages in terms of progression: stage I is called simple fatty liver; stage II denotes steatohepatitis, about 10% of which progressed to stage III; and stage III corresponds to fatty liver fibrosis and cirrhosis. Fatty liver is the second largest liver disease after hepatitis in China, and the incidence rate is still rising. Fatty liver patients are more common than hepatitis patients in Europe and America, and it is also one of the top ten common causes of death. In short, fatty liver has become a serious threat to human life and health, but medicaments for preventing and treating fatty liver still cannot meet clinical needs.

It has been reported that cholic acid derivatives have certain effects in the treatment of fatty liver. Norursodeoxycholic acid (norUDCA) has been reported to have the effect of preventing and treating NAFLA (Journal of Hepatology, 2010, 52: S304), a good therapeutic effect in cholestatic cirrhosis (Journal of Hepatology, 2017, 67:549), and also show a good protective effect in liver fibrosis models induced by thioacetamide (TAA). HTD1801 is a candidate drug molecule targeting AFLA and primary sclerosing cholangitis (PSC), with a structure as shown below, and the compound is composed of ursodeoxycholic acid and berberine. However, the therapeutic effects of these compounds on fatty liver still need to be further improved.

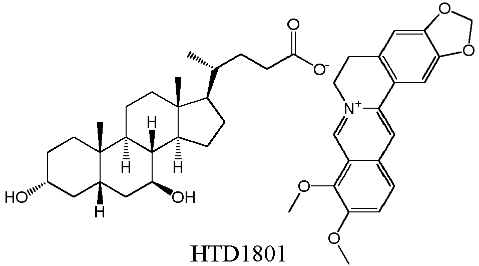

The inventors of the present invention have found in previous studies (see Chinese patent application number CN2021110940333) that the nor-ursodeoxycholic berberine salt composed of norUDCA and berberine salt can not only significantly reduce alanine aminotransferase (ALT), glutamic oxaloacetic transaminase (AST), and alkaline phosphatase (ALP), but also have a significant improvement effect on liver pathological damage (such as hepatic steatosis). The therapeutic effect of nor-ursodeoxycholic berberine salt on fatty liver, liver fibrosis, and cirrhosis is superior to the sum of the effects produced by individual norUDCA and berberine hydrochloride; the therapeutic effect of nor-ursodeoxycholic berberine salt on fatty liver, liver fibrosis, and cirrhosis is significantly better than that of HTD1801.

Solid chemical drugs are an important form for clinical application, and their content level is the main aspect of drug quality control. The basic requirement for high-quality solid chemical drugs is to have good stability. Therefore, when studying the polymorphism of solid chemical drugs, the stability of the crystalline form is one of the important study aspects. To investigate the stability of various crystalline solid substances, it is not only necessary to observe the stability of the active pharmaceutical ingredients (API) during storage, but also to observe the crystal stability under various conditions such as temperature, humidity, light, and pressure. Solid substances with good crystal stability should be selected as raw materials for development of new drug, to ensure that the drug does not undergo crystal transformation during preparation, transportation, storage, and preservation, which will affect the quality of the drug.

Therefore, it is of great significance to develop a highly stable crystalline form for the solid drug of nor-ursodeoxycholic berberine salt.

### Content of the invention

The object of the present invention is to provide a crystalline form of high-stability nor-ursodeoxycholic berberine salt, and a preparation method therefor.

The present invention provides a crystalline form of nor-ursodeoxycholic berberine salt, and the structure is as represented by formula I:

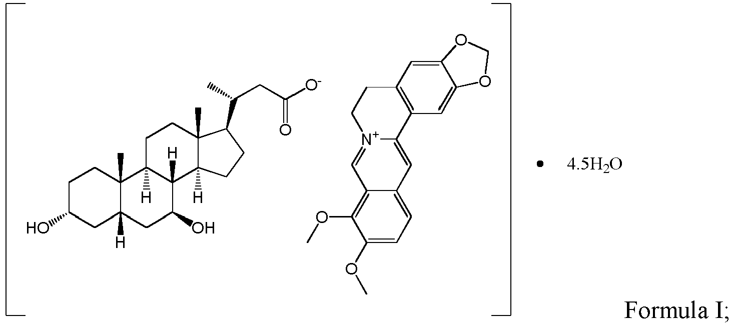

In the powder X-ray diffraction pattern of the crystalline form of nor-ursodeoxycholic berberine salt, the characteristic peaks were centered at diffraction angles (2*θ*) of 3.54±0.2°, 7.13±0.2°, 13.14±0.2°, 15.95±0.2°, 16.40±0.2°, 18.64±0.2°, 24.90±0.2°, 25.76±0.2°, and 26.30±0.2°.

Further, in the powder X-ray diffraction pattern of the crystalline form of nor-ursodeoxycholic berberine salt, the characteristic peaks were centered at diffraction angles (2*θ*) of 3.54±0.2°, 7.13±0.2°, 7.78±0.2°, 8.38±0.2°, 12.45±0.2°, 13.14±0.2°, 14.30±0.2°, 14.66±0.2°, 15.22±0.2°, 15.95±0.2°, 16.40±0.2°, 16.80±0.2°, 17.70±0.2°, 18.64±0.2°, 19.74±0.2°, 21.25±0.2°, 21.96±0.2°, 24.90±0.2°, 25.76±0.2°, 26.30±0.2°, and 28.00±0.2°.

Further, in the powder X-ray diffraction pattern of the crystalline form of nor-ursodeoxycholic berberine salt, the characteristic peaks were centered at diffraction angles (2*θ*) of 3.54±0.2°, 7.13±0.2°, 7.78±0.2°, 8.38±0.2°, 10.45±0.2°, 10.72±0.2°, 11.72±0.2°, 12.45±0.2°, 13.14±0.2°, 13.47±0.2°, 14.30±0.2°, 14.66±0.2°, 15.22±0.2°, 15.95±0.2°, 16.40±0.2°, 16.80±0.2°, 17.30±0.2°, 17.70±0.2°, 18.64±0.2°, 18.94±0.2°, 19.74±0.2°, 20.26±0.2°, 20.55±0.2°, 21.25±0.2°, 21.96±0.2°, 22.81±0.2°, 23.62±0.2°, 24.90±0.2°, 25.76±0.2°, 26.30±0.2°, 27.20±0.2°, 28.00±0.2°, 28.75±0.2°, 29.56±0.2°, 30.67±0.2°, 31.53±0.2°, 32.34±0.2°, 32.90±0.2°, and 34.04±0.2°.

Further, in the powder X-ray diffraction pattern of the crystalline form of nor-ursodeoxycholic berberine salt, the relative intensity of characteristic peaks at diffraction angles (2*θ*) is:

| Diffraction angles (2θ) | Relative intensity |
|---|---|
| 3.54±0.2° | 749 |
| 7.13±0.2° | 963 |
| 7.78±0.2° | 300 |
| 8.38±0.2° | 298 |
| 10.45±0.2° | 100 |
| 10.72±0.2° | 87 |
| 11.72±0.2° | 108 |
| 12.45±0.2° | 552 |
| 13.14±0.2° | 929 |
| 13.47±0.2° | 158 |
| 14.30±0.2° | 542 |
| 14.66±0.2° | 369 |
| 15.22±0.2° | 254 |
| 15.95±0.2° | 781 |
| 16.40±0.2° | 960 |
| 16.80±0.2° | 504 |
| 17.30±0.2° | 56 |
| 17.70±0.2° | 258 |
| 18.64±0.2° | 791 |
| 18.94±0.2° | 124 |
| 19.74±0.2° | 374 |
| 20.26±0.2° | 134 |
| 20.55±0.2° | 132 |
| 21.25±0.2° | 269 |
| 21.96±0.2° | 268 |
| 22.81±0.2° | 62 |
| 23.62±0.2° | 87 |
| 24.90±0.2° | 922 |
| 25.76±0.2° | 912 |
| 26.30±0.2° | 621 |
| 27.20±0.2° | 84 |
| 28.00±0.2° | 240 |
| 28.75±0.2° | 74 |
| 29.56±0.2° | 122 |
| 30.67±0.2° | 56 |
| 31.53±0.2° | 59 |
| 32.34±0.2° | 120 |
| 32.90±0.2° | 135 |
| 34.04±0.2° | 76 |

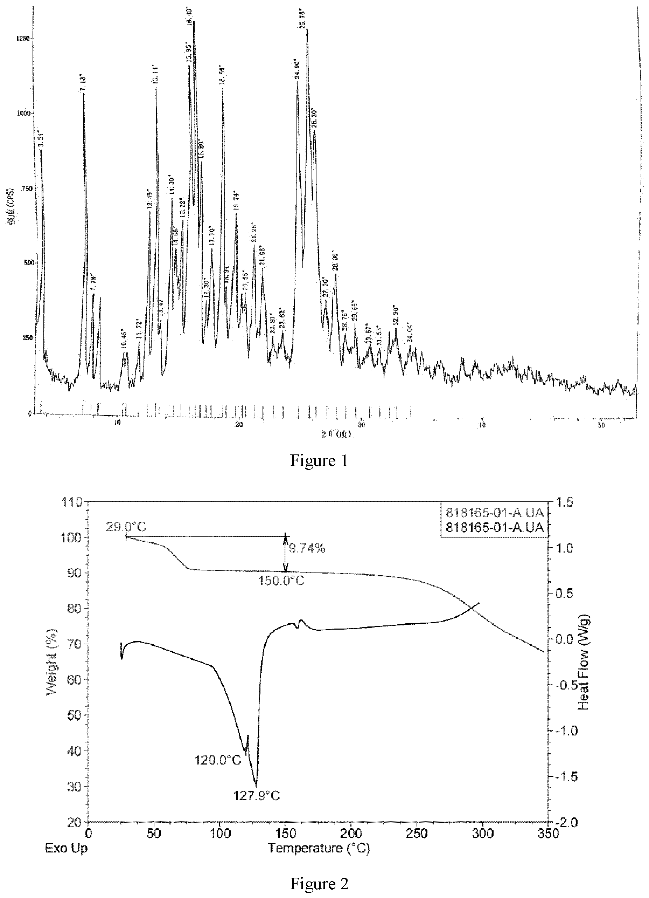

Further, the powder X-ray diffraction pattern of the crystalline form of nor-ursodeoxycholic berberine salt is shown in Figure 1.

Further, the asymmetric unit structure of the crystalline form of nor-ursodeoxycholic berberine salt comprises two nor-ursodeoxycholic acid anions, two berberine cations, and nine water molecules.

The present invention also provides a method for preparation of the crystalline form of nor-ursodeoxycholic berberine salt mentioned above, which comprises method 1 or method 2;

Method 1: The nor-ursodeoxycholic berberine salt is dissolved in a normal solvent, and then allowed to stand in a counter solvent gas environment, to obtain the crystalline form; at least one of the normal solvent and the counter solvent contains water; the structure of the nor-ursodeoxycholic berberine salt is as represented by formula 1-1:

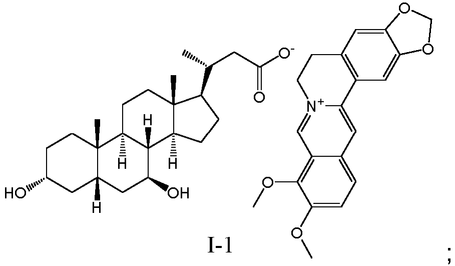

Method 2: Berberine hydrochloride or a hydrate thereof is dissolved in hot water at 50-100 °C, to obtain an aqueous solution of berberine hydrochloride; norUDCA is dissolved in an alkaline aqueous solution, and then added to the aqueous solution of berberine hydrochloride, followed by cooling to room temperature, to obtain the crystalline form.

Further, in method 1, the normal solvent is a polar organic solvent or a mixture of a polar organic solvent and water, and the polar organic solvent is selected from the group consisting of acetonitrile, dimethyl sulfoxide, N-methylpyrrolidone, methanol, ethanol, n-propanol, isopropanol, and trifluoroethanol, and a mixture thereof; the anti-solvent is selected from the group consisting of trichloromethane, tetrahydrofuran, n-pentane, ethyl acetate, isopropyl acetate, methyl tert-butyl ether, acetone, water, and a mixture thereof;
in method 2, the temperature of the hot water is 70-90 °C; the molar ratio of berberine hydrochloride or a hydrate thereof to nor-ursodeoxycholic acid is 1:(0.5-2); the alkaline aqueous solution is an aqueous solution of alkali, which is selected from the group consisting of sodium hydroxide, sodium carbonate, sodium bicarbonate, and a mixture thereof.

Further, in method 1, the normal solvent is n-propanol, and the counter solvent is water;
in method 2, the temperature of the hot water is 80 °C; the molar ratio of berberine hydrochloride or a hydrate thereof to norUDCA is 1: 1; the alkali is sodium hydroxide, and the aqueous solution of the alkali is an aqueous NaOH solution, with a mass fraction of 0.05% to 50%.

Further, in method 1, the standing temperature is room temperature, and the standing time is 3 days; the mass/volume ratio of nor-ursodeoxycholic berberine salt to the normal solvent is 40 mg/mL.

Further, in method 2, the mass/volume ratio of berberine hydrochloride or a hydrate thereof to the hot water is 1:(80-120) g/mL, the mass/volume ratio of norUDCA to the alkaline aqueous solution is 1:(10-30) g/mL, and the volume ratio of the hot water to the alkaline aqueous solution is (4-6): 1; preferably, the mass/volume ratio of berberine hydrochloride or a hydrate thereof to the hot water is 1: 100 g/mL, the mass/volume ratio of norUDCA to the alkaline aqueous solution is 1:20 g/mL, wherein the alkaline aqueous solution is an aqueous NaOH solution with a mass fraction of 0.5%, and the volume ratio of the hot water to the alkaline aqueous solution is 5:1.

The present invention also provides a medicament for the prevention and/or treatment of liver disease, and the medicament is a preparation prepared from the above crystalline form as the active ingredient, in combination with pharmaceutically acceptable excipients.

Further, the preparation is a solid preparation, and is preferably tablets, capsules, pills, pellets or granules.

The present invention also provides the use of the above crystalline form in the manufacture of medicaments for the prevention and/or treatment of liver disease.

In the present invention, room temperature means 25 ± 5 °C.

In the present invention, the crystalline form of nor-ursodeoxycholic berberine salt described in the above technical solution is named crystalline form A of nor-ursodeoxycholic berberine salt.

The present invention discloses crystalline form A of nor-ursodeoxycholic berberine salt, wherein the asymmetric unit of crystalline form A is composed of two nor-ursodeoxycholic acid anions (-1 valence state), two berberine cations (+1 valence state), and nine crystalline water molecules. Compared with other crystalline forms of nor-ursodeoxycholic berberine salts, crystalline form A has better physical stability, chemical stability, grinding stability, and pressure stability, and has good application prospects in the preparation of high-quality solid chemical drugs.

The method for preparing crystalline form A of nor-ursodeoxycholic berberine salt provided in the present invention is simple, and the raw materials are readily available, indicating that the method is suitable for scale-up production.

Obviously, based on the above content of the present invention, according to the common technical knowledge and the conventional means in the field, other various modifications, alternations, or changes can further be made, without department from the above basic technical spirits.

By following description of specific examples, the above content of the present invention is further illustrated. But it should not be construed that the scope of the above subject matter of the present invention is limited to the following examples. The techniques realized based on the above content of the present invention are all within the scope of the present invention.

### Figures

Figure 1 shows the XRPD pattern of crystalline form A of nor-ursodeoxycholic berberine salt;
Figure 2 shows the TGA/DSC graph of crystalline form A;
Figure 3 shows ¹H NMR spectrum of crystalline form A;
Figure 4 shows comparison of XRPD patterns of crystalline form A obtained in Example 1 and that obtained in Example 2;
Figure 5 shows the XRPD pattern of crystalline form B of nor-ursodeoxycholic berberine salt;
Figure 6 shows the TGA/DSC graph of crystalline form B;
Figure 7 shows ¹H NMR spectrum of crystalline form B;
Figure 8 shows the XRPD pattern of crystalline form C of nor-ursodeoxycholic berberine salt;
Figure 9 shows the TGA/DSC graph of crystalline form C;
Figure 10 shows ¹H NMR spectrum of crystalline form C;
Figure 11 shows the XRPD pattern of crystalline form D of nor-ursodeoxycholic berberine salt;
Figure 12 shows the TGA/DSC graph of crystalline form D;
Figure 13 shows ¹H NMR spectrum of crystalline form D;
Figure 14 shows the XRPD pattern of crystalline form F of nor-ursodeoxycholic berberine salt;
Figure 15 shows the TGA/DSC graph of crystalline form F;
Figure 16 shows ¹H NMR spectrum of crystalline form F;
Figure 17 shows comparison of XRPD patterns of crystalline form G and crystalline form A of nor-ursodeoxycholic berberine salt;
Figure 18 shows the XRPD pattern of crystalline form H of nor-ursodeoxycholic berberine salt;
Figure 19 shows the XRPD pattern of single crystal obtained by cultivation;
Figure 20 shows the structure of asymmetric unit for single crystal of crystalline form A;
Figure 21 shows the schematic diagram of molecular stacking structure for single crystal of crystalline form A;
Figure 22 shows the XRPD overlay for competition of crystalline forms A, C, and D in suspension;
Figure 23 shows the Dynamic Vapor Sorption (DVS) graph of crystal form A;
Figure 24 shows the XRPD overlay of crystalline form A before and after DVS testing;
Figure 25 shows the XRPD overlay of crystalline form A sample before and after stability testing;
Figure 26 shows the XRPD overlay of crystalline form D sample before and after stability testing;
Figure 27 shows the XRPD overlay of crystalline form A before and after grinding stability testing;
Figure 28 shows the XRPD overlay of crystalline form D before and after grinding stability testing;
Figure 29 shows the XRPD overlay of crystalline form A before and after pressure stability testing;
Figure 30 shows the XRPD overlay of crystalline form D before and after pressure stability testing.

### Examples

The raw materials and equipment used in the specific examples of the present invention are all known products obtained by purchasing those commercially available.

### Example 1. Preparation of crystalline form A of nor-ursodeoxycholic berberine salt

### 1. Preparation of starting materials of nor-ursodeoxycholic berberine salt

The starting material of nor-ursodeoxycholic berberine salt was prepared according to the method described in Example 1 of the Chinese patent application number CN2021110940333. The specific procedures are shown in Scheme 1:

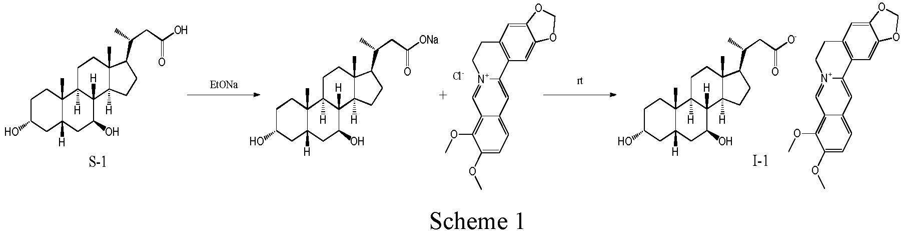

0.79 g of berberine hydrochloride (2.12 mmol) was dissolved in 4 mL of methanol, to which was added sodium sulfate to dry for 60 min, and then the solution was filtered, followed by addition of sodium sulfate for later use. Moreover, 0.80 g of norUDCA (S-1, 2.11 mmol) was dissolved in 4 mL of ethanol, to which was added 0.15 g of sodium ethoxide (2.20 mmol), and then the resultant solution was stirred at room temperature for 10 min, and concentrated to dry. The obtained solid was dissolved in 4 mL of methanol, and then the solution was dropped into the methanol solution of berberine hydrochloride. The resultant solution was stirred at room temperature for 1 h, and filtered. The filtrate was concentrated to dryness. The crude product was dispersed in ethyl acetate and crystallized. The crystal was dried to obtain compound 1-1 (i.e. the starting material of nor-ursodeoxycholic berberine salt, 1.18 g), with a yield of 78.1%.

¹HNMR (DMSO-d₆, 400 MHz): *δ* 9.88 (s, 1H), 8.92 (s, 1H), 8.20 (d, *J* = 9.1 Hz, 1H), 8.00 (d, *J =* 9.1 Hz, 1H), 7.79 (s, 1H), 7.08 (s, 1H), 4.96-4.88 (m, 2H), 4.09 (s, 3H), 4.06 (s, 3H), 3.79 (s, 1H), 3.33-3.23 (m, 2H), 3.23-3.16 (m, 2H), 2.09-2.00 (m, 1H), 1.96-1.88 (m, 1H), 1.84-1.54 (m, 6H), 1.58-1.25 (m, 9H), 1.25-0.91 (m, 7H), 0.91-0.88 (m, 3H), 0.88-0.82 (m, 6H), 0.61 (s, 3H).

### 2. Preparation of crystalline form A of nor-ursodeoxycholic berberine salt

20 mg starting material of nor-ursodeoxycholic berberine salt was placed in a clean glass vial (4 mL), and then 0.5 mL of n-propanol was added as the normal solvent to dissolve the sample. The 4 mL glass vial was opened and placed in a 20 mL glass bottle containing 3 mL of water as the counter solvent. The 20 mL glass bottle was covered and sealed, and then placed at room temperature for 3 days, to obtain crystalline form A of nor-ursodeoxycholic berberine salt.

### Characterization of crystalline form A:

The powder X-ray diffraction (XRPD) pattern of crystalline form A is shown in Figure 1. In Figure 1, crystalline form A exhibits characteristic peaks at the following diffraction angles (2*θ*): 3.54°, 7.13°, 7.78°, 8.38°, 10.45°, 10.72°, 11.72°, 12.45°, 13.14°, 13.47°, 14.30°, 14.66°, 15.22°, 15.95°, 16.40°, 16.80°, 17.30°, 17.70°, 18.64°, 18.94°, 19.74°, 20.26°, 20.55°, 21.25°, 21.96°, 22.81°, 23.62°, 24.90°, 25.76°, 26.30°, 27.20°, 28.00°, 28.75°, 29.56°, 30.67°, 31.53°, 32.34°, 32.90°, 34.04°.

In XRPD pattern of crystalline form A, the relative intensity of the characteristic peaks at following diffraction angles (2*θ*) is:

| Diffraction angle (2*θ*) | Relative intensity |
|---|---|
| 3.54 | 749 |
| 7.13 | 963 |
| 7.78 | 300 |
| 8.38 | 298 |
| 10.45 | 100 |
| 10.72 | 87 |
| 11.72 | 108 |
| 12.45 | 552 |
| 13.14 | 929 |
| 13.47 | 158 |
| 14.30 | 542 |
| 14.66 | 369 |
| 15.22 | 254 |
| 15.95 | 781 |
| 16.40 | 960 |
| 16.80 | 504 |
| 17.30 | 56 |
| 17.70 | 258 |
| 18.64 | 791 |
| 18.94 | 124 |
| 19.74 | 374 |
| 20.26 | 134 |
| 20.55 | 132 |
| 21.25 | 269 |
| 21.96 | 268 |
| 22.81 | 62 |
| 23.62 | 87 |
| 24.90 | 922 |
| 25.76 | 912 |
| 26.30 | 621 |
| 27.20 | 84 |
| 28.00 | 240 |
| 28.75 | 74 |
| 29.56 | 122 |
| 30.67 | 56 |
| 31.53 | 59 |
| 32.34 | 120 |
| 32.90 | 135 |
| 34.04 | 76 |

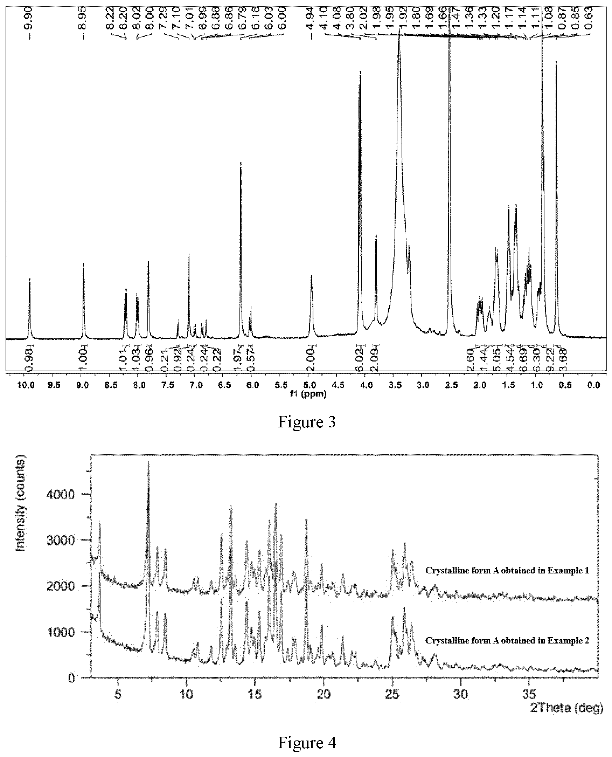

The TGA/DSC graph of crystalline form A is shown in Figure 2, and the ¹H NMR spectrum is shown in Figure 3. The TGA graph indicates that crystalline form A is characterized by showing a weight loss of 9.74% before 100 °C, and a water peak was observed in the ¹H NMR spectrum, indicating that crystalline form A is a crystal form of a hydrate; the DSC graph shows that crystalline form A is characterized by having two endotherm peaks (peak temperature) at 120.0 °C and 127.9 °C.

### Example 2. Preparation of crystalline form A of nor-ursodeoxycholic berberine salt

3.00 g of commercially available berberine hydrochloride hydrate (labeled with a molecular weight of 371.81, 8.07 mmol) was mixed with 300 mL of water, and then heated to 80 °C to dissolve, so as to obtain an aqueous solution of berberine hydrochloride. Moreover, 0.3 g of sodium hydroxide was dissolved in 60 mL of water, and then 3.05 g of norUDCA (8.06 mmol) was dissolved in the aqueous solution of sodium hydroxide. The resultant solution was slowly added to the aqueous solution of berberine hydrochloride, stirred at 80 °C for 30 min, and then cooled to room temperature, followed by filtering and drying, to obtain crystalline form A of nor-ursodeoxycholic berberine salt as yellow solids.

Comparison of the XRPD spectra of the crystalline forms obtained in Example 2 and Example 1 is shown in Figure 4, and as shown, the positions and intensities of characteristic peaks for the two crystalline form samples at the 2*θ* diffraction angles are the same, indicating that the crystalline form obtained in Example 2 is also form A.

The following is the method for preparing the control crystalline forms.

### Comparative example 1. Preparation of crystalline form B of nor-ursodeoxycholic berberine salt

150 mg of crystalline form A obtained in Example 2 was mixed with 2 mL of ethanol/n-hexane (with a volume ratio of 1:5) solution, and then stirred at 50 °C for 3 days, to obtain crystalline form B.

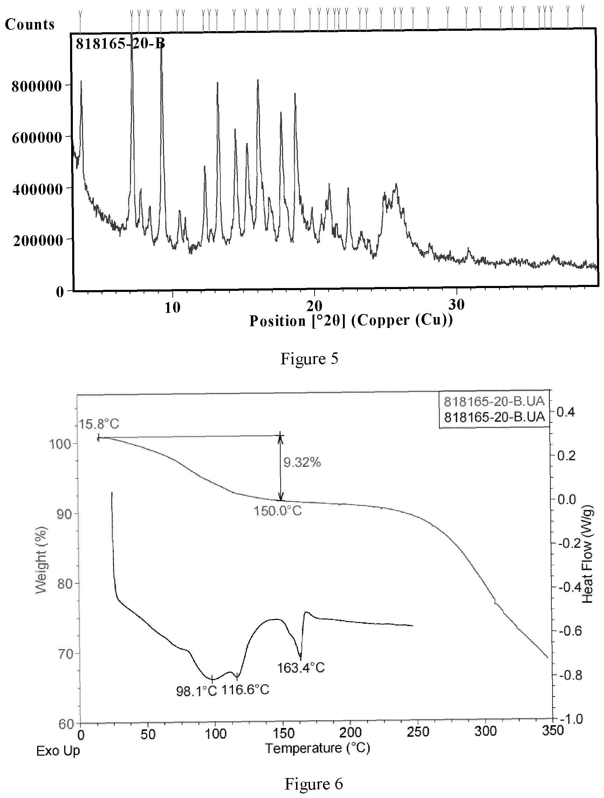

The XRPD pattern of crystalline form B is shown in Figure 5. Crystalline form B has the characteristic peaks at following diffraction angles (2*θ*): 3.68°, 7.28°, 7.81°, 8.44°, 9.36°, 10.52°, 10.92°, 12.36°, 13.31°, 14.52°, 15.31°, 16.15°, 16.85°, 17.73°, 18.75°, 21.13°, 22.42°, 25.80°.

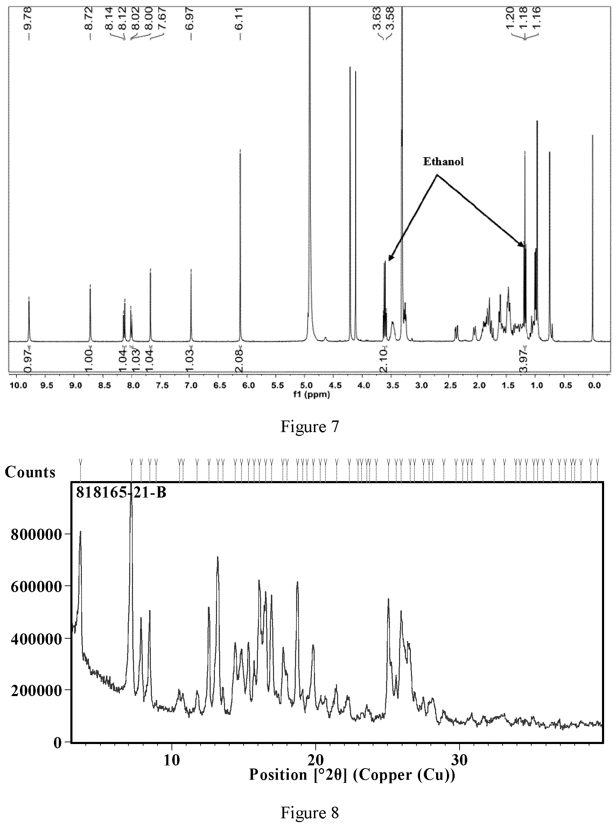

The TGA/DSC graph of crystalline form B is shown in Figure 6, and the ¹H NMR spectrum is shown in Figure 7. The TGA graph shows that crystalline form B is characterized by having a weight loss of 9.32% before 150 °C, and the ¹H NMR spectrum indicates that the residual solvent is ethanol, corresponding to a weight loss of 6.4%. The DSC graph shows that crystalline type B is characterized by having three endotherm peaks (peak temperature) at 98.1, 116.3, and 163.4 °C.

### Comparative example 2. Preparation of crystalline form C of nor-ursodeoxycholic berberine salt

150 mg of crystalline form A obtained in Example 2 was mixed with 2 mL of n-propanol/isopropylbenzene (with a volume ratio of 1:4) solution, and then stirred at room temperature for 3 days, to obtain crystalline form C.

The XRPD pattern of crystalline form C is shown in Figure 8. Crystalline form C has the characteristic peaks at following diffraction angles (2*θ*): 3.64°, 7.19°, 7.86°, 8.44°, 10.78°, 11.73°, 12.58°, 13.22°, 13.54°, 14.40°, 14.82°, 15.32°, 16.08°, 16.54°, 16.92°, 17.72°, 18.73°, 19.85°, 25.05°, 25.94°, 26.56°.

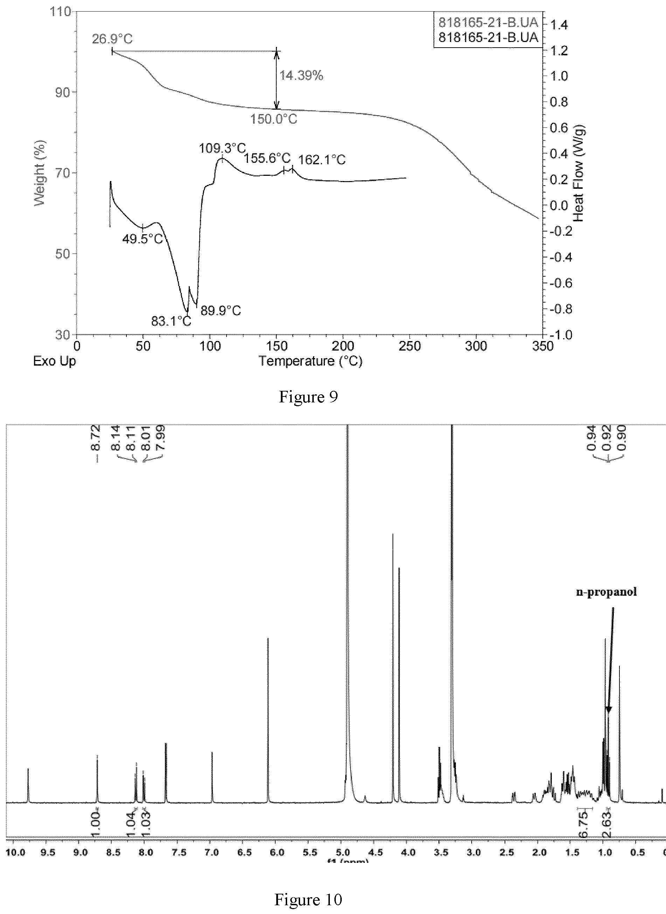

The TGA/DSC graph of crystalline form C is shown in Figure 9, and the ¹H NMR spectrum is shown in Figure 10. The TGA graph shows that crystalline form C is characterized by having a weight loss of 14.4% before 150 °C, and the ¹H NMR spectrum indicates that the residual solvent is n-propanol, corresponding to a weight loss of 6.9%. The DSC graph shows that crystalline type C is characterized by having three endotherm peaks (peak temperature) at 49.5, 83.1, and 89.9 °C, together with three exotherm peaks (peak temperature) at 109.3, 155.6, and 162.1 °C.

### Comparative example 3. Preparation of crystalline form D of nor-ursodeoxycholic berberine salt

150 mg of crystalline form A obtained in Example 2 was mixed with 2 mL of acetonitrile, and then stirred at 50 °C for 3 days, to obtain crystalline form D.

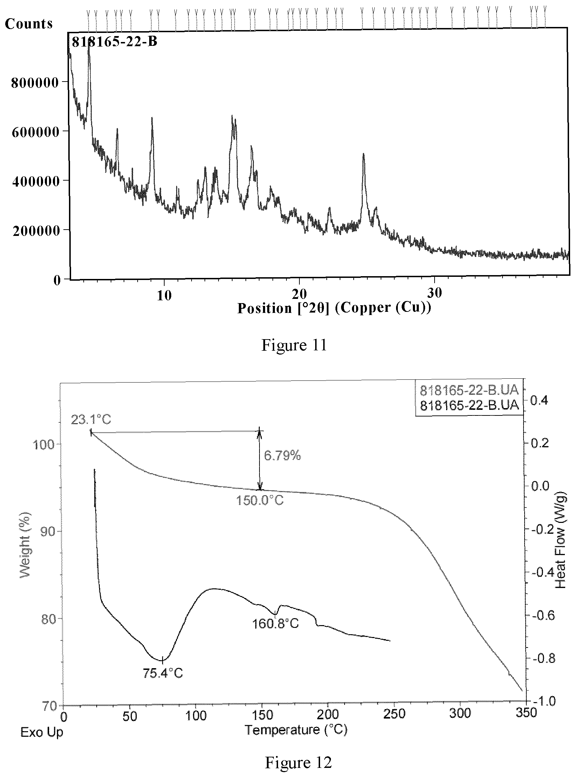

The XRPD pattern of crystalline form D is shown in Figure 11, which comprises the characteristic peaks at following diffraction angles (2*θ*): 4.55°, 6.57°, 9.20°, 12.56°, 13.11°, 13.89°, 15.11°, 15.36°, 16.53°, 16.87°, 17.95°, 24.82°, 25.64°.

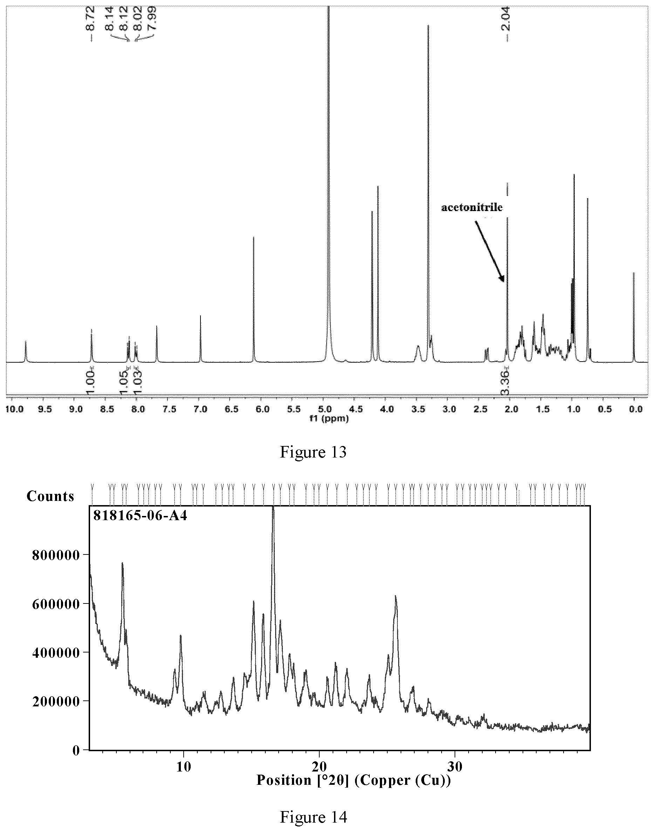

The TGA/DSC results of crystalline form D are shown in Figure 12, and the ¹H NMR spectrum is shown in Figure 13. The TGA results showed that crystalline form D is characterized by having a weight loss of 6.79% before 150 °C, while the ¹H NMR spectrum indicates that the residual solvent is acetonitrile, corresponding to a weight loss of 3.9%. The DSC results indicated that crystalline type D is characterized by having two endotherm peaks (peak temperature) at 75.4 and 160.8 °C.

### Comparative example 4. Preparation of crystalline form F of nor-ursodeoxycholic berberine salt

150 mg of crystalline form A obtained in Example 2 was subjected to gas-solid permeation under the atmosphere of 15 mL dimethyl sulfoxide, to obtain crystalline form F.

The XRPD pattern of crystalline form F is shown in Figure 14, in which the characteristic peaks were centered at following diffraction angles (2*θ*): 5.48°, 5.73°, 9.30°, 9.75°, 15.13°, 15.85°, 16.60°, 17.10°, 17.77°, 18.10°, 18.98°, 21.28°, 22.06°, 23.68°, 25.09°, 25.63°.

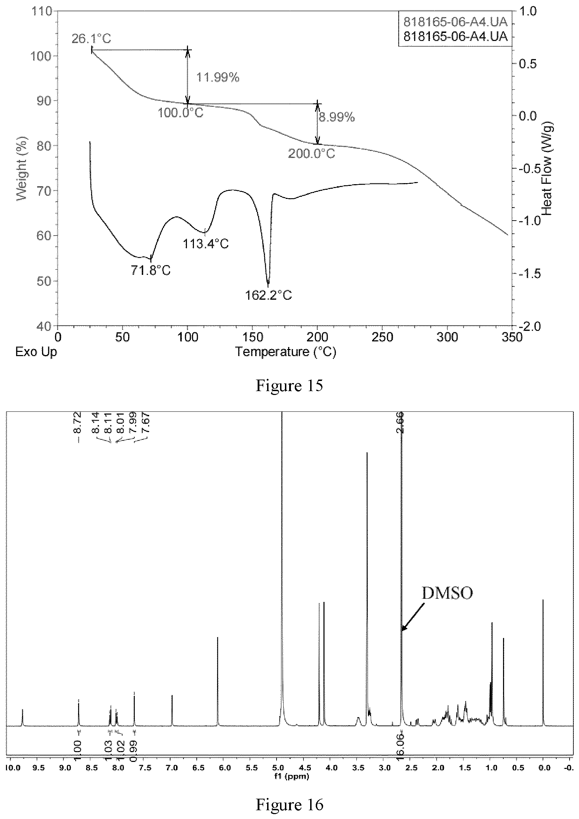

The TGA/DSC results of crystalline form F are shown in Figure 15, and the ¹H NMR spectrum is shown in Figure 16. The TGA results indicated that crystalline form F is characterized by having a weight loss of 20.98% before 200 °C, while the ¹H NMR spectrum confirms that the residual solvent is DMSO, corresponding to a weight loss of 22.7%. The DSC results indicated that crystalline form F is characterized by having three endotherm peaks (peak temperature) at 71.8, 113.4 and 162.2 °C.

### Comparative example 5. Preparation of crystalline form G of nor-ursodeoxycholic berberine salt

150 mg of crystalline form A obtained in Example 2 was purged with nitrogen for 1 h, to obtain crystal form G

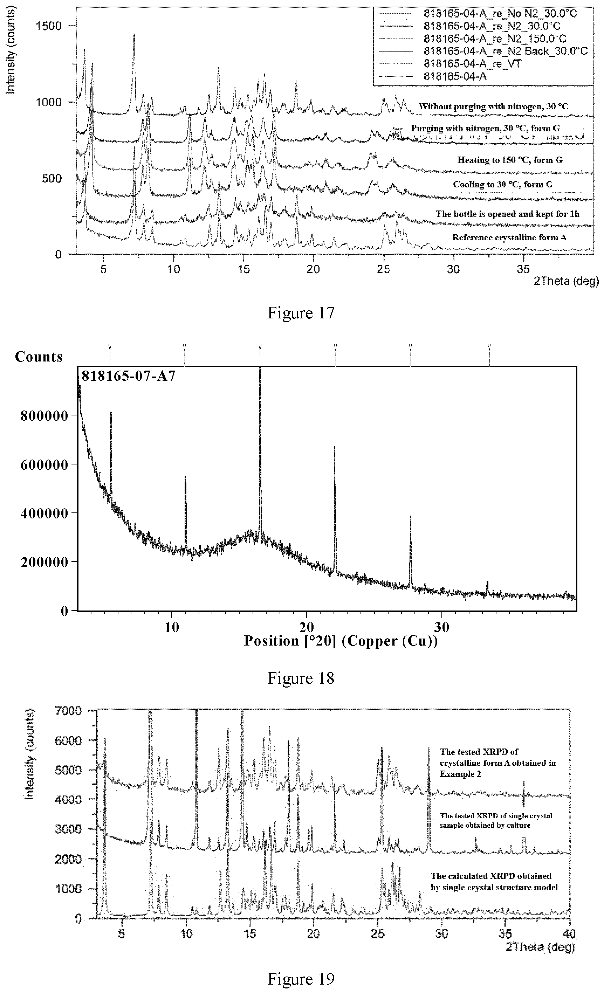

Comparison of XRPD spectra for crystalline form G and crystalline form A is shown in Figure 17.

Crystalline form G was anhydrous, and was not changed when heated to 150 °C and then cooled to 30 °C. However, after opening the cover and kept for 1 h, form G turned back into form A.

### Comparative example 6. Preparation of crystalline form H of nor-ursodeoxycholic berberine salt

150 mg of crystalline form A obtained in Example 2 was dissolved in 3 mL of N-methylpyrrolidone, and then placed in a large bottle containing water, to obtain crystalline form H after gas-liquid permeation.

The XRPD pattern of crystalline form H is shown in Figure 18, in which the characteristic peaks were centered at following diffraction angles (2*θ*): 5.42°, 10.94°, 16.53°, 22.13°, 27.68°, 33.50°.

The beneficial effects of the present invention were demonstrated by following experimental examples.

Experimental example 1: Culture and analysis of crystalline form A of nor-ursodeoxycholic berberine salt

The starting material of nor-ursodeoxycholic berberine salt (10.0 mg) was placed in a clean glass vial (4 mL), to which was added 0.8 mL mixed solution of acetonitrile and water (with a volume ratio of 1:5) as normal solvent to dissolve the material. The 4 mL glass vial was sealed, and a small hole was made on the cap. Then, the vial was transferred into a 20 mL glass bottle containing 3 mL of trichloromethane as counter solvent, followed by covering and sealing the 20 mL glass bottle, which was placed in a 40 °C biochemical incubator for gas-liquid diffusion culture, to obtain blade-like single crystals.

The obtained single crystal was determined as crystalline form A by XRPD detection, and the pattern is shown in Figure 19.

Structural characterization of single crystal indicated that the asymmetric unit of crystalline form A was composed of two nor-ursodeoxycholic acid anions (-1 valence state), two berberine cations (+1 valence state), and nine crystal water molecules. The structural formula as represented by formula 1 could be used to show half nonahydrate of the ionic salt composed of nor-ursodeoxycholic acid and berberine:

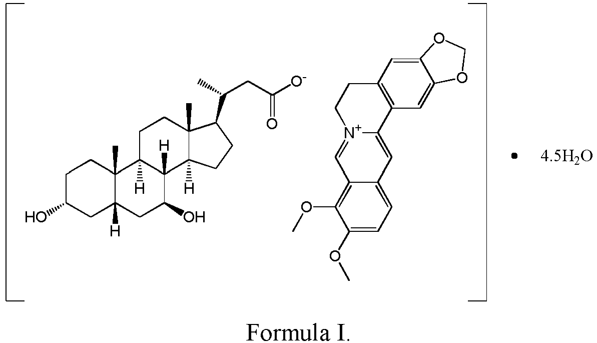

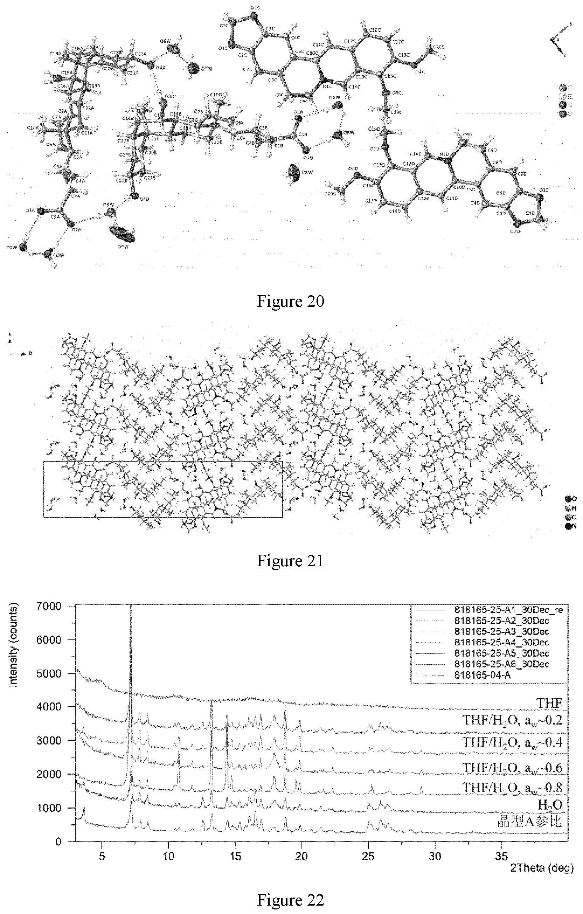

The asymmetric unit structure of crystalline form A is shown in Figure 20, and the schematic diagram of molecular stacking structure is shown in Figure 21. The crystallographic data are recorded in Table 1:

**Table 1. Crystallographic data of crystalline form A of nor-ursodeoxycholic berberine salt.**

| | | |
|---|---|---|
| Empirical formula | C₄₃H₅₅NO₈ • 4.5H₂O | |
| Temperature | 139.0 K | |
| Wavelength | Ga/Kα (λ=1.34138 Å) | |
| Crystal system, space group | Monoclinic, *P*2₁ | |
| Unit cell dimensions | a = 7.0503(7) Å | α = 90° |
| | b = 49.048(5) Å | β = 92.524(3)° |
| | c = 11.6103(12) Å | γ = 90° |
| Volume | 4011.0(7) Å³ | |
| Z, Calculated density | 4, 1.316 g/cm³ | |
| 2 Theta range for data collection | 3.134 to 116.444° | |
| Reflections collected/Independent reflections | 113061/17008 [Rᵢₙₜ= 0.0479, R_{sigma} = 0.0281] | |
| Completeness | 99.93% | |
| Data / restraints / parameters | 17008/1/1059 | |
| Goodness-of-fit on F2 | 1.018 | |
| Final R indices [I>=2sigma(I)] | R₁ = 0.0436, wR₂ = 0.1222 | |
| Largest diff. peak and hole | 0.91/-0.67Å⁻³ | |
| Flack parameter | 0.07(3) | |

### Experimental example 2: Competition experiment for crystalline forms A, C, and D in suspension

Six solvent systems were selected for the competition experiment in suspension, namely tetrahydrofuran, tetrahydrofuran/water (0.98:0.02, aw~0.2), ethanol/water (0.96:0.04, aw~0.4), ethanol/water (0.92:0.08, aw~0.6), ethanol/water (0.87:0.13, aw~0.8), and water. The starting material obtained in step 1 of Example 1 was added to the corresponding volume of solvent, and then allowed to suspend under stirring at room temperature for 2 h. The suspension was filtered with a 0.4 µm PTFE filter membrane to obtain a saturated solution. About 3 mg samples of crystalline forms A, C, and D were separately weighed and transferred to HPLC vials, to which was added 1 mL of saturated solution, and then the mixture was allowed to suspend under stirring at room temperature. After 8 days, the suspension was filtered to obtain the solid, and the change in crystalline form was identified by XRPD analysis.

The XRPD stacking results for competition in suspension are shown in Figure 22. The results indicated that when a_{w} was 0, amorphous solid was obtained in the competition suspension; while when a_{w} was 0.2-1, crystalline form A was obtained. That suggested that during competition in suspension, crystalline forms C and D would transition to form A.

### Experimental example 3: Hygroscopicity test

The mass change percentage of crystalline form A was tested using dynamic vapor sorption (DVS) under constant temperature conditions of 25 °C and various humidity conditions, with a humidity change of 30% RH to 95% RH to 0% RH to 95% RH.

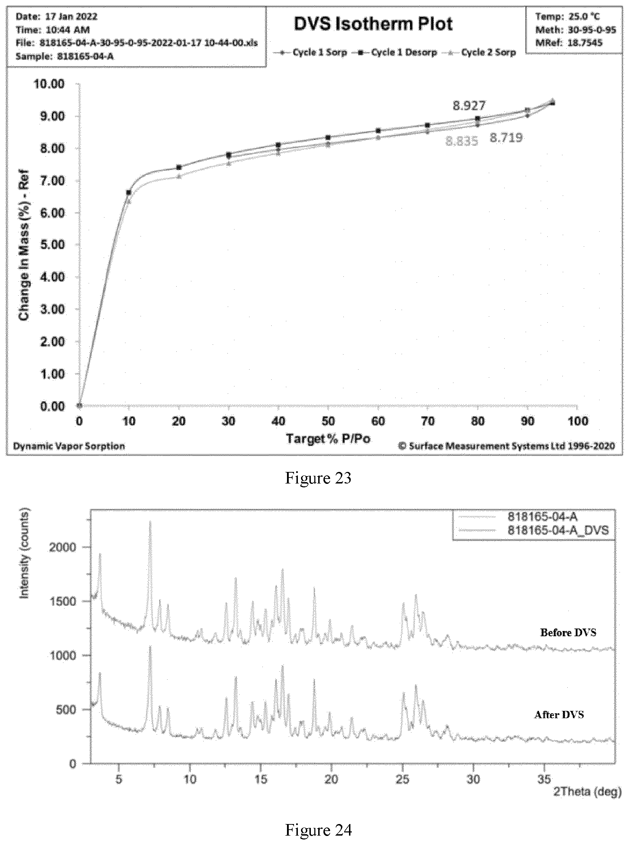

As shown in Figure 23, the sample of crystalline form A rapidly absorbed water during the relative humidity range of 0% to 10%, while slowly absorbed water after the relative humidity reached 10%; when the humidity was 80% RH, the water adsorption rate was 8.7%. After DVS testing, the sample was subjected to XRPD detection, and the results are shown in Figure 24, indicating that crystalline form A did not change before and after DVS testing. Crystalline form A had good physical stability.

### Experimental example 4: Stability testing

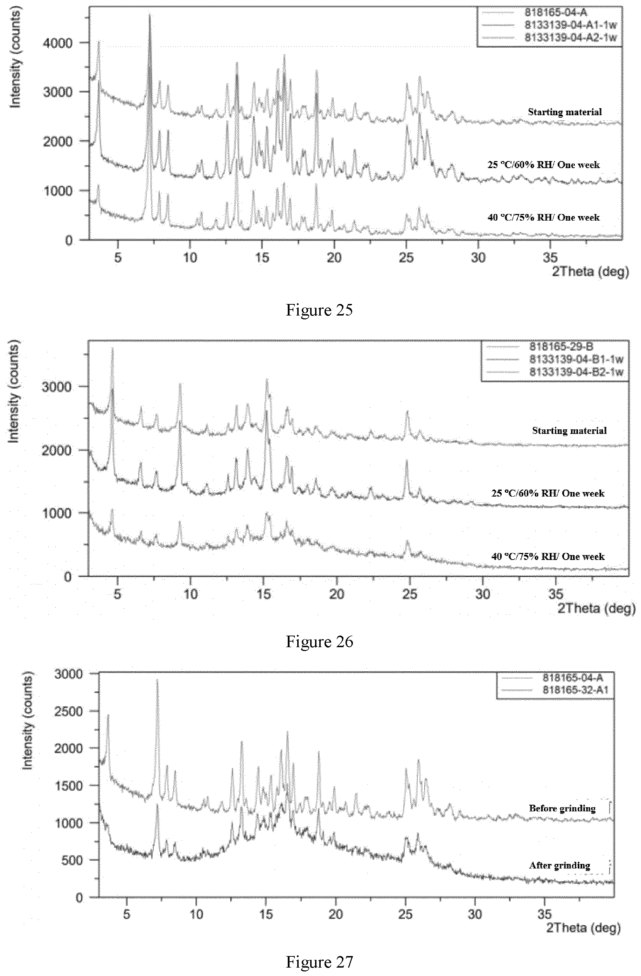

The samples of crystalline forms A and D were stored in an opened bottle for one week under 25 °C/60% RH and 40 °C/75% RH conditions, respectively. The physical and chemical stability of the samples was detected by XRPD and HPLC. The test results are shown in Table 2. The XRPD results of crystalline form A are shown in Figure 25, while the XRPD results of crystalline form D are shown in Figure 26.

**Table 2. Solid stability results of crystalline forms A and D.**

| Initial crystalline form | Time point | Conditions | Crystalline form | Purity (area ) | Relative purity percentage (vs initial purity) |
|---|---|---|---|---|---|
| Crystalline form A | Initial | - | Crystalline form A | 99.26% | -- |
| | One week | 25°C/60%RH | Crystalline form A | 99.24% | 100.0% |
| | One week | 40°C/75%RH | Crystalline form A | 99.23% | 100.0% |
| Crystalline form D | Initial | - | Crystalline form D | 98.61% | -- |
| | One week | 25°C/60%RH | Crystalline form D | 98.24% | 99.63% |
| | One week | 40°C/75%RH | Crystalline form D | 97.99% | 99.37% |

The results indicated that the purity of crystalline form A was not changed significantly under the above two conditions, and thus its chemical stability was good; moreover, the crystal structure was not changed, and thus the physical stability was good. After being placed at 25 °C/60% RH for one week, the purity of crystalline form D decreased by 0.37%; after being placed at 40 °C/75% RH for one week, the purity decreased by 0.62%. Although the crystalline form was not obviously changed, the crystallinity decreased, and the sample turned dark brown. In summary, the physical and chemical stability of crystalline form A is superior to that of form D.

### Experimental example 5: Grinding stability testing

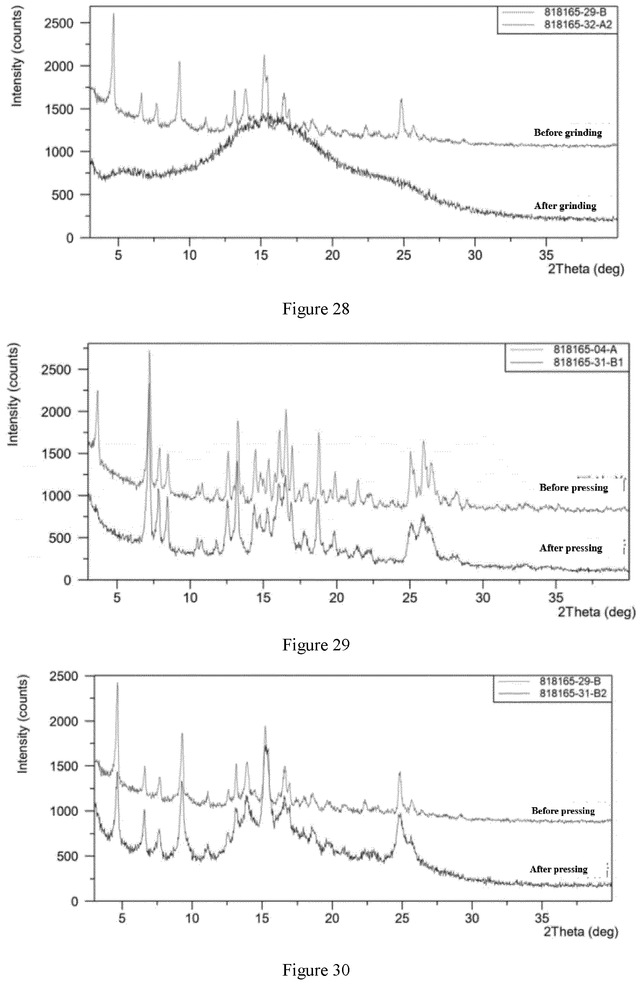

50 mg samples of crystalline form A and crystalline form D were manually ground, and then detected by XRPD. The test results of crystalline form A are shown in Figure 27, while the test results of crystalline form D are shown in Figure 28.

The results indicated that after grinding, crystalline form A remained unchanged, and the crystallinity decreased. Crystalline form D transformed into amorphous after grinding. In summary, the grinding stability of crystalline form A is significantly better than that of form D.

### Experimental example 6: Pressure stability testing

100 mg samples of crystalline forms A and D were respectively added to 6 mg of round flat punch, and then pressed into circular tablets using 10 kN pressure. The loading mass (ML) and tablet mass (MT) were recorded, and then the formula ma = (ML-MT)/ML×100% was used to calculate the mass percentage (ma) of the powder adhesive to the punch; the pressed tablets were kept in a dryer for 24 h for elastic recovery. After elastic recovery, the diameter (D) and thickness (t) of the tablet were measured using a vernier caliper. The radial crushing strength (hardness, H) was measured using a tablet hardness tester, and the XRPD pattern of the crushed sample was also tested. The results of crystalline form A are shown in Figure 29, while crystalline form D are shown in Figure 30. The tensile strength of the powder was calculated under different hardness using the formula T=2H/πDt. The results are shown in following Table 3:

**Table 3. Results of pressure stability testing for crystalline forms A and D.**

| Parameters | Crystalline form A | Crystalline form D |
|---|---|---|
| Tablet diameter D (mm ) | 6.02 | 6.02 |
| Tablet thickness t (mm ) | 2.90 | 2.90 |
| Hardness H (N ) | 65.37 | 46.31 |
| Tensile strength T (MPa ) | 2.38 | 1.69 |
| Loading mass (mg ) | 104.9 | 99.9 |
| Tablet mass (mg ) | 103.2 | 96.9 |
| Adhesion amount (mg ) | 1.7 | 3.3 |
| Adhesion percentage % | 1.6 | 3.3 |

The results indicated that under the same pressing conditions, compared to crystalline form D, crystalline form A can be used to produce tablets with higher hardness, greater tensile strength, and less adhesion, which is more conducive to obtaining stable preparations; under the same pressing conditions, both crystalline form A and crystalline form D remained unchanged.

In summary, the present invention provided a crystalline form of high-stability nor-ursodeoxycholic berberine salt, and a preparation method therefor. In the powder X-ray diffraction pattern of the crystalline form, the characteristic peaks were centered at diffraction angles (2*θ*) of 3.54±0.2°, 7.13±0.2°, 13.14±0.2°, 15.95±0.2°, 16.40±0.2°, 18.64±0.2°, 24.90±0.2°, 25.76±0.2°, and 26.30±0.2°. Compared with other crystalline forms of nor-ursodeoxycholic berberine salts, this crystalline form had better physical stability, chemical stability, grinding stability, and pressure stability, and had good application prospects in the preparation of high-quality solid chemical drugs.

Although the above examples provided a detailed description of the present invention, they are merely a part of the examples according to the present invention, not all of them. Other examples can also be obtained without inventive step based on the present examples, all of which fall within the protection scope of the present invention.

## Claims

1. A crystalline form of nor-ursodeoxycholic berberine salt, **characterized in that** the crystal structure of the nor-ursodeoxycholic berberine salt is as represented by formula I: 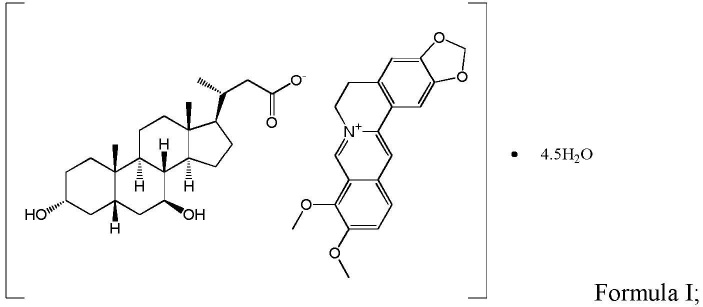 in the powder X-ray diffraction pattern of the crystalline form of nor-ursodeoxycholic berberine salt, the characteristic peaks were centered at diffraction angles (2θ) of 3.54±0.2°, 7.13±0.2°, 13.14±0.2°, 15.95±0.2°, 16.40±0.2°, 18.64±0.2°, 24.90±0.2°, 25.76±0.2°, and 26.30±0.2°.

2. The crystalline form of nor-ursodeoxycholic berberine salt according to claim 1, **characterized in that** in the powder X-ray diffraction pattern of the crystalline form of nor-ursodeoxycholic berberine salt, the characteristic peaks were centered at diffraction angles (2θ) of 3.54±0.2°, 7.13±0.2°, 7.78±0.2°, 8.38±0.2°, 12.45±0.2°, 13.14±0.2°, 14.30±0.2°, 14.66±0.2°, 15.22±0.2°, 15.95±0.2°, 16.40±0.2°, 16.80±0.2°, 17.70±0.2°, 18.64±0.2°, 19.74±0.2°, 21.25±0.2°, 21.96±0.2°, 24.90±0.2°, 25.76±0.2°, 26.30±0.2°, and 28.00±0.2°.

3. The crystalline form of nor-ursodeoxycholic berberine salt according to claim 2, **characterized in that** in the powder X-ray diffraction pattern of the crystalline form of nor-ursodeoxycholic berberine salt, the characteristic peaks were centered at diffraction angles (2θ) of 3.54±0.2°, 7.13±0.2°, 7.78±0.2°, 8.38±0.2°, 10.45±0.2°, 10.72±0.2°, 11.72±0.2°, 12.45±0.2°, 13.14±0.2°, 13.47±0.2°, 14.30±0.2°, 14.66±0.2°, 15.22±0.2°, 15.95±0.2°, 16.40±0.2°, 16.80±0.2°, 17.30±0.2°, 17.70±0.2°, 18.64±0.2°, 18.94±0.2°, 19.74±0.2°, 20.26±0.2°, 20.55±0.2°, 21.25±0.2°, 21.96±0.2°, 22.81±0.2°, 23.62±0.2°, 24.90±0.2°, 25.76±0.2°, 26.30±0.2°, 27.20±0.2°, 28.00±0.2°, 28.75±0.2°, 29.56±0.2°, 30.67±0.2°, 31.53±0.2°, 32.34±0.2°, 32.90±0.2°, and 34.04±0.2°.

4. The crystalline form of nor-ursodeoxycholic berberine salt according to claim 3, **characterized in that** in the powder X-ray diffraction pattern of the crystalline form of nor-ursodeoxycholic berberine salt, the relative intensity of characteristic peaks at diffraction angles (2θ) is:
| Diffraction angle (2θ) | Relative intensity |
|---|---|
| 3.54±0.2° | 749 |
| 7.13±0.2° | 963 |
| 7.78±0.2° | 300 |
| 8.38±0.2° | 298 |
| 10.45±0.2° | 100 |
| 10.72±0.2° | 87 |
| 11.72±0.2° | 108 |
| 12.45±0.2° | 552 |
| 13.14±0.2° | 929 |
| 13.47±0.2° | 158 |
| 14.30±0.2° | 542 |
| 14.66±0.2° | 369 |
| 15.22±0.2° | 254 |
| 15.95±0.2° | 781 |
| 16.40±0.2° | 960 |
| 16.80±0.2° | 504 |
| 17.30±0.2° | 56 |
| 17.70±0.2° | 258 |
| 18.64±0.2° | 791 |
| 18.94±0.2° | 124 |
| 19.74±0.2° | 374 |
| 20.26±0.2° | 134 |
| 20.55±0.2° | 132 |
| 21.25±0.2° | 269 |
| 21.96±0.2° | 268 |
| 22.81±0.2° | 62 |
| 23.62±0.2° | 87 |
| 24.90±0.2° | 922 |
| 25.76±0.2° | 912 |
| 26.30±0.2° | 621 |
| 27.20±0.2° | 84 |
| 28.00±0.2° | 240 |
| 28.75±0.2° | 74 |
| 29.56±0.2° | 122 |
| 30.67±0.2° | 56 |
| 31.53±0.2° | 59 |
| 32.34±0.2° | 120 |
| 32.90±0.2° | 135 |
| 34.04±0.2° | 76 |

5. The crystalline form of nor-ursodeoxycholic berberine salt according to claim 4, **characterized in that** the powder X-ray diffraction pattern of the crystalline form of nor-ursodeoxycholic berberine salt is shown in Figure 1.

6. The crystalline form of nor-ursodeoxycholic berberine salt according to any one of claims 1 to 5, **characterized in that** the asymmetric unit structure of the crystalline form of nor-ursodeoxycholic berberine salt comprises two nor-ursodeoxycholic acid anions, two berberine cations, and nine water molecules.

7. A method for preparation of the crystalline form of nor-ursodeoxycholic berberine salt according to any one of claims 1 to 6, **characterized in that** the method comprises method 1 or method 2;
Method 1: The nor-ursodeoxycholic berberine salt is dissolved in a normal solvent, and then allowed to stand in a counter solvent gas environment, to obtain the crystalline form of nor-ursodeoxycholic berberine salt; at least one of the normal solvent and the counter solvent contains water; the structure of the nor-ursodeoxycholic berberine salt is as represented by formula 1-1: 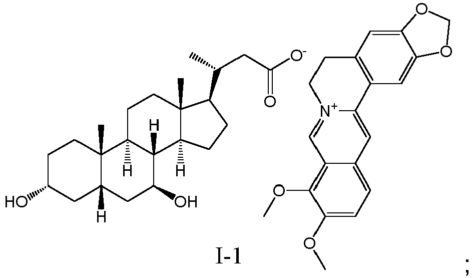
Method 2: Berberine hydrochloride or its hydrate is dissolved in hot water at 50-100 °C, to obtain an aqueous solution of berberine hydrochloride; the nor-ursodeoxycholic acid is dissolved in an alkaline aqueous solution, and then added to the aqueous solution of berberine hydrochloride, followed by cooling to room temperature, to obtain the crystalline form of nor-ursodeoxycholic berberine salt.

8. The method according to claim 7, **characterized in that** in method 1, the normal solvent is a polar organic solvent or a mixture of a polar organic solvent and water, and the polar organic solvent is selected from the group consisting of acetonitrile, dimethyl sulfoxide, N-methylpyrrolidone, methanol, ethanol, n-propanol, isopropanol, and trifluoroethanol, and a mixture thereof; the counter solvent is selected from the group consisting of trichloromethane, tetrahydrofuran, n-pentane, ethyl acetate, isopropyl acetate, methyl tert-butyl ether, acetone, water, and a mixture thereof;
in method 2, the temperature of the hot water is 70-90 °C; the molar ratio of berberine hydrochloride or a hydrate thereof to nor-ursodeoxycholic acid is 1:(0.5-2); the alkaline aqueous solution is an aqueous solution of alkali, which is selected from the group consisting of sodium hydroxide, sodium carbonate, sodium bicarbonate, and a mixture thereof.

9. The method according to claim 8, **characterized in that** in method 1, the normal solvent is n-propanol, and the counter solvent is water;
in method 2, the temperature of the hot water is 80 °C; the molar ratio of berberine hydrochloride or a hydrate thereof to nor-ursodeoxycholic acid is 1: 1; the alkali is sodium hydroxide.

10. The method according to claim 7, **characterized in that** in method 1, the standing temperature is room temperature, and the standing time is 3 days; the mass/volume ratio of nor-ursodeoxycholic berberine salt to the normal solvent is 40 mg/mL.

11. The method according to claim 7, **characterized in that** in method 2, the mass/volume ratio of berberine hydrochloride or a hydrate thereof to the hot water is 1:(80-120) g/mL, the mass/volume ratio of nor-ursodeoxycholic acid to the alkaline aqueous solution is 1:(10-30) g/mL, and the volume ratio of the hot water to the alkaline aqueous solution is (4-6): 1.

12. The method according to claim 11, **characterized in that** in method 2, the mass/volume ratio of berberine hydrochloride or a hydrate thereof to the hot water is 1: 100 g/mL, the mass/volume ratio of nor-ursodeoxycholic acid to the alkaline aqueous solution is 1:20 g/mL, and the volume ratio of the hot water to the alkaline aqueous solution is 5:1.

13. A medicament for the prevention and/or treatment of liver disease, **characterized in that** the medicament is a preparation prepared from the crystalline form according to any one of claims 1-6 as the active ingredient, in combination with pharmaceutically acceptable excipients.

14. The medicament according to claim 13, **characterized in that** the preparation is a solid preparation.

15. The medicament according to claim 14, **characterized in that** the solid preparation is tablets, capsules, pills, pellets or granules.

16. The crystalline form of nor-ursodeoxycholic berberine salt according to any one of claims 1 to 6 for use in the manufacture of medicaments for the prevention and/or treatment of liver disease.
